# EUROPEAN PATENT SPECIFICATION

(11) **EP 2 200 545 B1**
(45) Date of publication and mention of the grant of the patent: **18.09.2013**
(21) Application number: 08840541.0
(22) Date of filing: 10.10.2008
(51) Int. Cl.: A61F 5/02

(54) **ADJUSTABLE POSTERIOR SPINAL ORTHOSIS**
VERSTELLBARE POSTERIORE WIRBELSÄULENORTHESE
ORTHÈSE VERTÉBRALE POSTÉRIEURE RÉGLABLE

(30) Priority: 17.10.2007 US 999308 P
(43) Date of publication of application: 30.06.2010
(73) Proprietor: Orthomerica Products, Inc., Orlando, FL 32810 (US)
(72) Inventor: SANDIFER, Alan, T., Casselberry FL 32707 (US); SCHWENN, Shannon, R., Deltona FL 32788 (US)
(74) Representative: Viering, Jentschura & Partner
(86) International application number: PCT/US2008/079592
(87) International publication number: WO 2009/052031

(56) References cited:
- WO-A1-99/65428
- WO-A1-2007/043079
- US-A- 1 581 791
- US-A- 2 828 737
- US-A- 3 945 376
- US-A- 5 548 843
- US-A1- 2005 067 816

## Description

### BACKGROUND OF THE INVENTION

### 1. Field of the Invention.

The present invention is directed to a lightweight spinal brace or othopraxis device for treatment of patients with upper back pain, strain, osteoporosis and compression fractures and more particularly, to an easily worn and adjustable extension compression posterior spinal orthosis for applying extension and compression forces in the treatment of spinal disorders.

### 2. Description of Related Art.

The prior art discloses various devices to provide an orthotic treatment to relieve pain and to provide compensation for various types of spinal disorders such as multiple compression fractures that may occur from osteoporosis and kythotic postural changes.

Frequently, these spinal braces have employed a triangular or three point force vector system that includes an anchor point centrally located across the chest of the user which can be provided by a sternal pad supported, for example, by a metal frame and a corresponding anchor point on the lower portion of the abdomen, provided by a pubic pad supported by a frame. Thus, two forward pressure points are provided on the front of the user and a centrally located adjustable lumbar pad is provided on the user's back, again adjustably fixed to a metal frame so that three force vectors can be applied to create a condition of hyperextension on the spinal column, while permitting some mobility to the user. An example can be seen in the Jewett brace shown in U.S. Patent No. 3,274,996.

Basically, this orthotic modality of treatment involves an anterior spinal hyperextension orthosis to create spaced anterior anchor stabilization points across the sternum and pubic areas with an adjustable force applied at an intermediate position on the user's back. Numerous variations of this treatment can be found, for example in U.S. Patent No. 5,599,287, U.S. Patent No. 6,190,343, U.S. Patent No. 6,790,191, U.S. Patent No. 6,010,472, U.S. Patent No. 5,674,187, and U.S. Patent No. 3,945,376. Other examples and variations can also be found in the orthodesic art.

An article in the American Journal of Physical Medicine and Rehabilitation "Effects of a New Spinal Orthosis of Posture, Trunk Strength And Quality of Life In Women With Postmenopausal Osteoporosis," Volume 83, No. 3, Pages 177-186, March 2004, described a study on the management of vertebra fractures caused by osteoporosis. A thoracolumbar orthosis disclosed in the study included a narrow back pad centered along the spine which could be workable as cold material to adjust to the particular patient, a system of belts, and an abdominal pad to apply lower, intermediate and upper forces on the back pad to mold it to the spinal region of the user. See U.S. Patent No. 6,063,047, U.S. Patent No. 2,828,737, U.S. Patent No. 1,755,641 and U.S. Patent No. 1,581,791.

There is still a need in the orthopedic field to provide an economical lightweight, comfortably wearing extension compression orthosis to treat mid spinal pathologies that does not require cumbersome strapping arrangements. Such a brace should be easily adjusted by the user to accommodate for various daily activity demands, to provide an improved, firm, lightweight posterior support structure that is easy to apply and that permits vector forces to be applied to the body by the user and to permit adjustments to match the size and configuration of the user.

The International Patent Application Publication No. WO 2007/043079 A1 discloses an orthopedic support or dorsal orthesis which can be used to support and block the spinal column of subjects with articular back problems or suffering from osteoporosis. That orthopedic support or dorsal orthesis comprises technical features which are described in the preamble of independent claim 1.
The International Patent Application Publication No. WO 99/65428 A1 discloses an orthotic device including an orthosis body adapted to be wrapped around the torso of a wearer of the device, the orthosis body having at least two segments in juxtaposed relationship.

### SUMMARY OF THE INVENTION

The present invention provides a spinal orthosis which is characterized by the technical features described in the characterizing portion of independent claim 1. Preferred embodiments of the present invention are described in the dependent claims.

The present invention provides a lightweight spinal orthosis that can be adjustably mounted, for example by an orthotist in an economical manner so that it can be easily placed on a user's body to apply extension and compression forces in the treatment of spinal disorders. As can be appreciated, various medical issues and spinal problems can occur frequently with aging. The present orthosis can be economically manufactured and easily placed on and removed from the user or patient, preferably similar to putting on a clothing jacket. A rigid posterior frame or spinal member can be formed from a stamped metal plate or from a molded plastic and can be further subjectively configured to meet the demands and requirements of the specific patient.

A pair of flexible strap members, attached adjacent an upper portion of the posterior spinal member, and attached adjacent a lower portion of the spinal member are provided on opposite lateral sides of the spinal member, and have a sufficient length to provide adjustable shoulder straps for the user. Thus, the shoulder straps can be conveniently adjusted to permit the user's arms to extend through the shoulder straps and then abdomen or waist closure pads are fastened together across the abdominal area of the patient and subsequently tighten to draw the flexible strap members against the patient's body.

The waist closure pads can comprise a pair of flexible abdomen support members, having for example, a connecting strap attached to respective smaller loops of the flexible strap members located below the loops for accommodating shoulder straps. The flexible abdomen support members can adhere together and connecting straps with pocketed finger tabs can be adjustably fastened across the surface of the flexible abdomen support members and each other to secure the spinal member on the user while pulling the flexible strap members.

The pair of flexible strap members can be removably attached adjacent an upper portion of the spinal member and permanently attached to a lower portion of the spinal member and adjustments can be easily made by an orthotist to both the flexible strap members and the connecting straps in order to size the spinal orthosis to a user.

The posterior spinal member is approximately of a length to extend across the user's thoracic to the coccyx of the user's spine. The posterior spinal member can have an elongated triangular configuration with apertures to reduce weight. The spinal member can be formed from a thin metal plate and bent to increase strength while configured to the user's spine. Alternatively, the spinal member can be molded from a plastic resin.

A replaceable resilient pad of a nap material on one side and an apertured texture to permit air flow can be removably attached to an internal surface of the posterior spine member for contacting the user's back.

### BRIEF DESCRIPTION OF THE DRAWINGS

The objects and features of the present invention, which are believed to be novel, are set forth with particularity in the appended claims. The present invention, both as to its organization and manner of operation, together with further objects and advantages, may best be understood by reference to the following description, taken in connection with the accompanying drawings.

Figure 1 is a front perspective view of a posterior spinal orthosis on a user;

Figure 2 is an elevated rear view of the spinal orthosis;

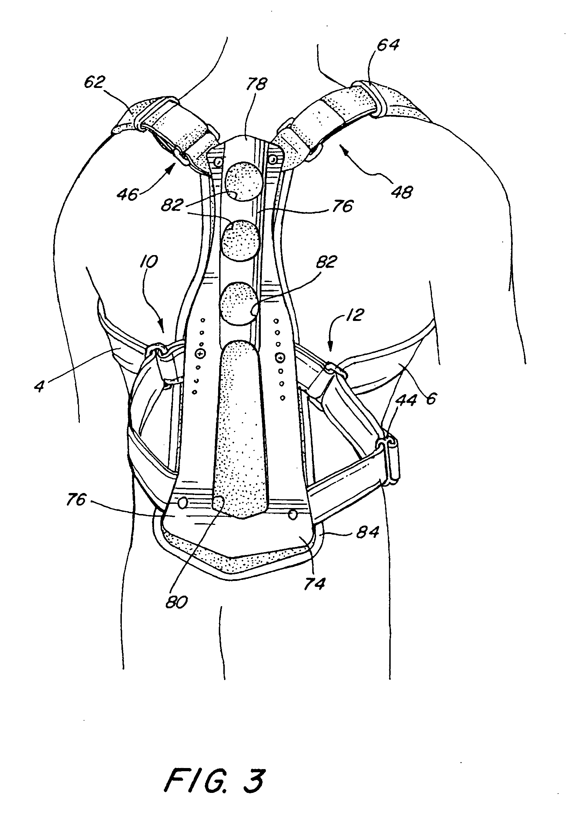

Figure 3 is a rear view of the orthosis on a user;

Figure 4 is a side view of the orthosis on a user;

Figure 5 is a partial side view of the side strapping configuration;

Figure 6 is a perspective view of a user beginning the donning of the orthosis by closing the abdomen support members;

Figure 7 shows the user tightening the spinal orthosis by placing his fingers in pocketed finger pull tabs at the end of tightening straps;

Figure 8 is a perspective view disclosing an adjustment of a tightening strap fitting on a user; and

Figure 9 is a perspective view disclosing an adjustment of flexible strap members for adjustment to a user.

### DETAILED DESCRIPTION OF THE PREFERRED EMBODIMENTS

Reference will now be made in detail to the preferred embodiments of the invention which set forth the best modes contemplated to carry out the invention, examples of which are illustrated in the accompanying drawings. While the invention will be described in conjunction with the preferred embodiments, it will be understood that they are not intended to limit the invention to these embodiments.

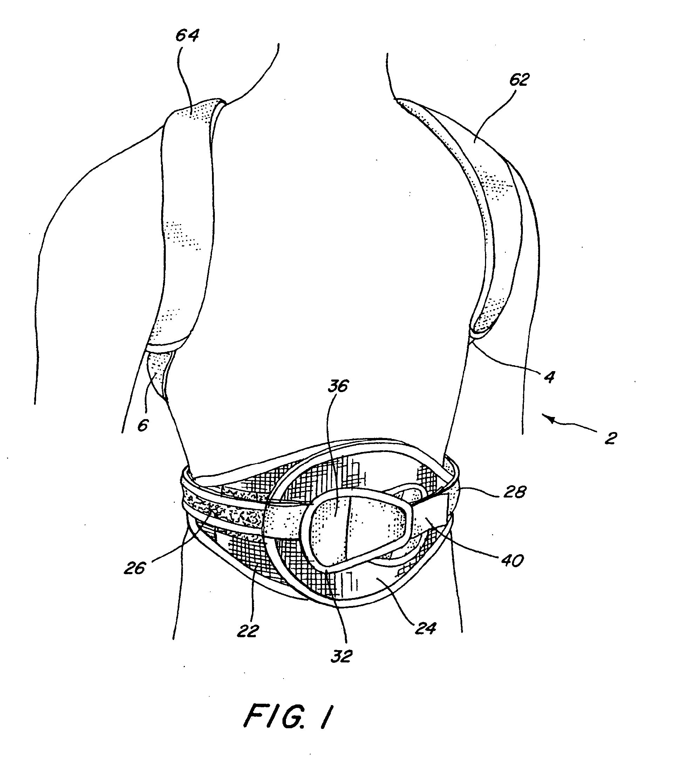

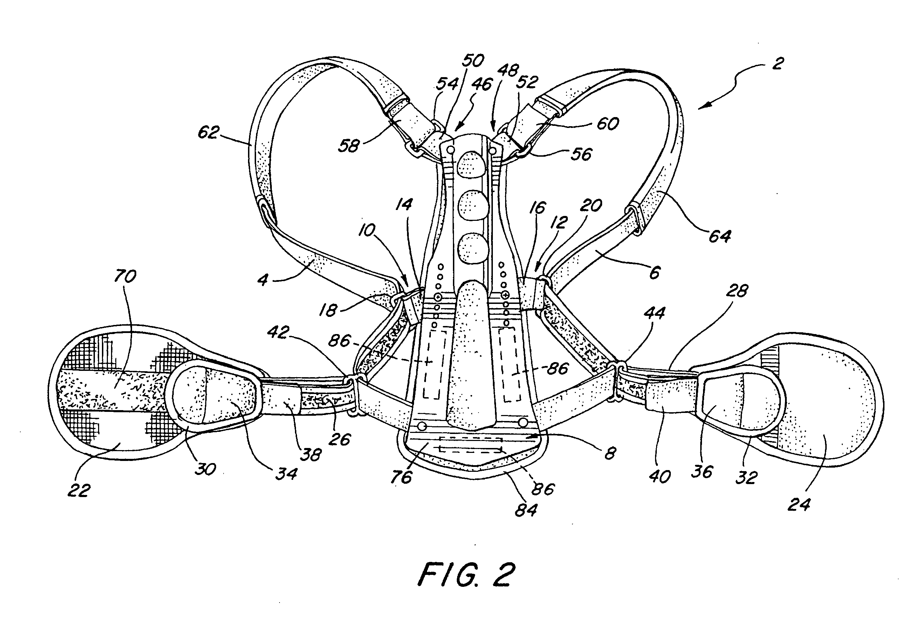

Referring to Figures 1 and 2 a spinal orthosis 2 has a posterior spinal member 8 with a frame member 74 either of a bent and curved metal plate or a molded plastic resin part.

A flexible resilient pad 84 is adhered on an internal surface of the spinal frame member 74 to provide a comfortable cushioned fit for the user. Elongated flexible strap members 4 and 6 are removably attached at a top portion of an upper edge of a curved neck 78 of the spinal frame member 74. Upper attachment members 46 and 48 of appropriate looped straps 50 and 52 are fastened by a screw or rivet as seen in Figure 2 adjacent the top of the curved neck 78. The respective straps 50 and 52 anchor an upper attachment loop 54 and 56, respectively that can also support spinal adjustment straps 58 and 60.

The elongated flexible strap members 4 and 6 can be removably fastened to the respective loops 54 and 56. The strap members 4 and 6 extend along the lateral sides of the spinal frame member 74. The curved neck 78 extends down to an intermediate portion of the spinal frame member 74 and intermediate attachment members 10 and 12 are mounted on either side, in a width direction, of the spinal frame member 74. Looping attachment straps 14 and 16 are appropriately fastened, for example, by rivets to the frame member 74 and capture attachment loops 18 and 20, respectively.

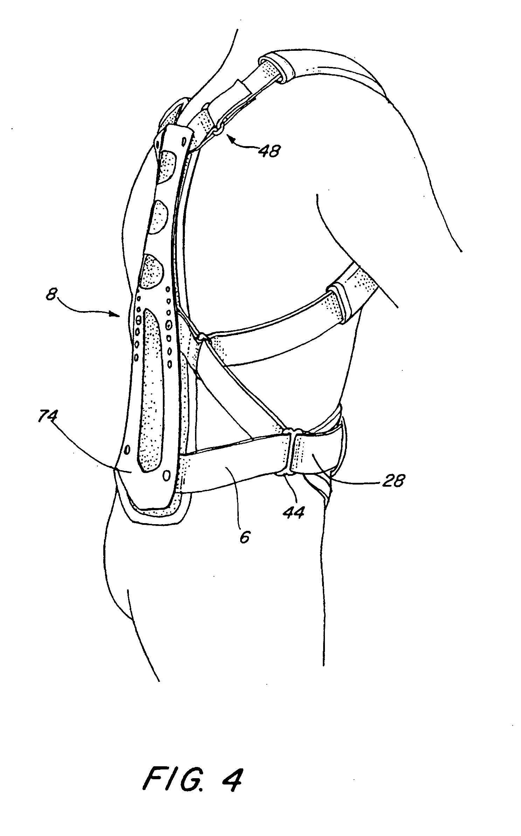

The flexible strap members 4 and 6 are journaled through the respective attachment loops 18 and 20 and are permanently fixed at a spinal triangular base 76 of the spinal frame member 74, for example by a rivet or other fastener, see Figures 2 and 4.

Each of the flexible strap members 4 and 6 are configured to provide upper major loops that function as shoulder straps for a user or patient. Tubular shoulder pads 62 and 64 are mounted on the respective strap members 4 and 6 within the upper major loops to cushion any force on the patient's shoulders. Minor lower loops of the strap members 4 and 6 are further created between the riveted ends of the respective flexible strap members 4 and 6 and the attachment loops 18 and 20, respectively to enable securing the posterior spinal member triangular base 78 against the user's lower back as shown in Figure 4.

Flexible padded abdominal support members 22 and 24 support connecting straps 26 and 28, respectively, that extend through respective double loops 42 and 44 on either side of the posterior spinal member 8. Thus, the abdominal support members 22 and 24 can slide with the connecting straps 26 and 28 along the minor loops formed by the flexible strap members 4 and 6, see Figures 2, 4 and 5.

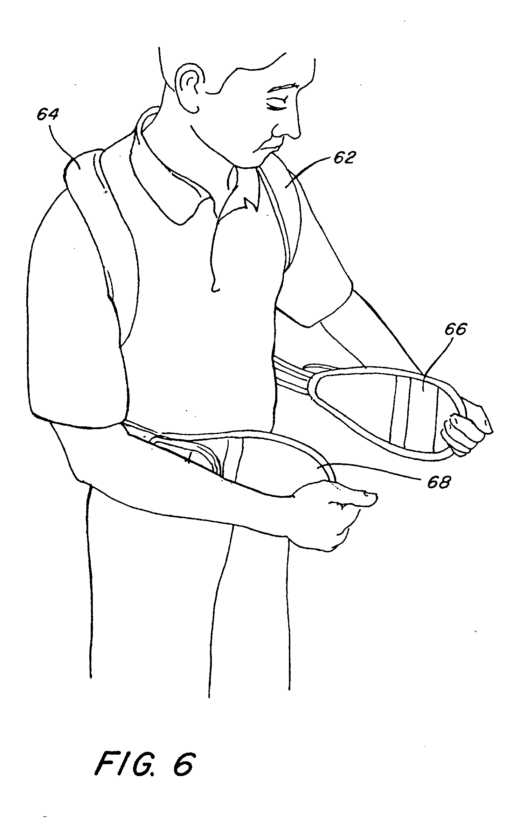

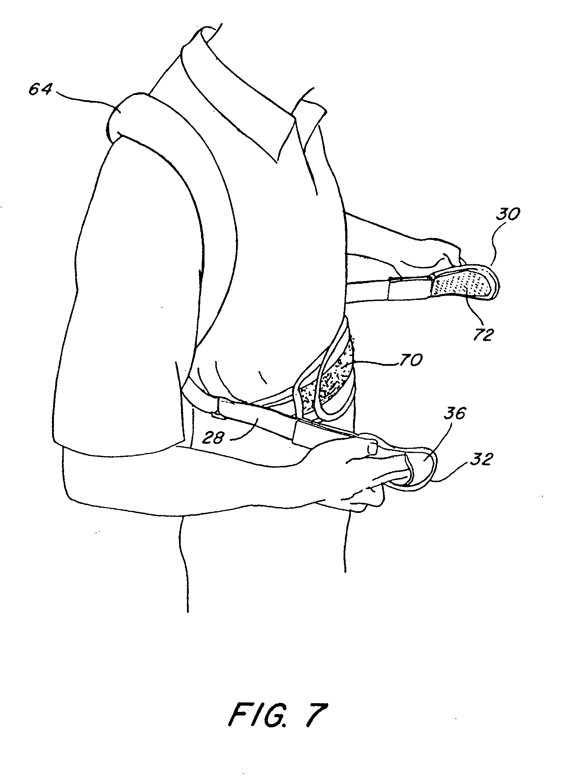

As can be seen in Figure 6, the respective abdomen support members 22 and 24 can be pulled and loosely fastened across an abdomen section of the user, as shown in Figure 7. An abdomen support swatch of hook material 66, for example of Velcro® can be located on an inner surface of each abdomen support member 22 and 24. Correspondingly, the outside surface of each support member 22 and 24 can have a nap fabric covering 68. As a result, a subjective fastening, in an overlapping manner, of the abdomen support members 22 and 24 can be performed by the user after the user's arms are inserted through the shoulder straps.

Additionally, as seen in Figure 7, fastening pads 30 and 32 with exterior pockets 34 and 36 respectively, of a size to receive one or more fingers, are attached at the other end of the respective connecting straps 26 and 28. Due to the size of the abdomen support members 22 and 24 and the fastening pads 30 and 32, the straps 26 and 28 are captured in one of the loops of the respective double loop members 42 and 44. The underside of each of the fastening pads 30 and 32 have a fastening hook material 72 capable of engaging the napped or looped fabric of the reinforced straps 26 and 28 and an abdomen swatch of nap material 70 on an exterior surface of the abdomen support member 22.

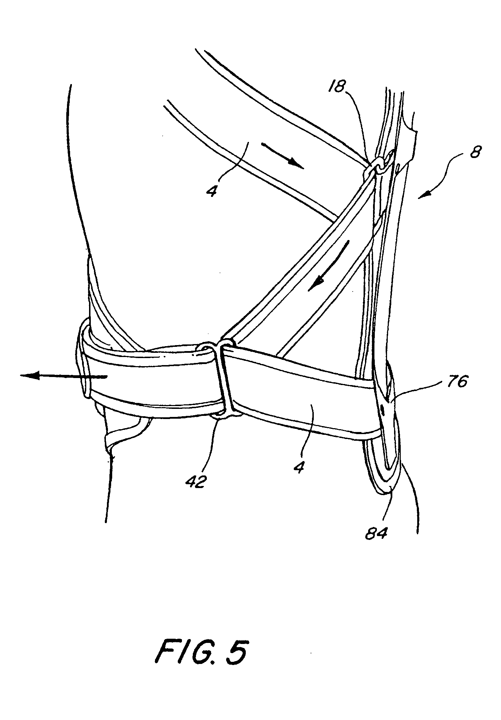

Thus, as seen in Figure 7, pulling the respective fastening pads 30 and 32 tightens the waist connecting straps 26 and 28 and pulls the smaller loops of the flexible strap members 4 and 6, and as seen in Figure 5, will also tighten the shoulder straps of flexible strap members 4 and 6 to pull the user's shoulders backwards for a snug fit against the posterior spinal member 8. The fastening pads 30 and 32 can adhere to each other and also the nap material of the abdomen support members 22 and 24.

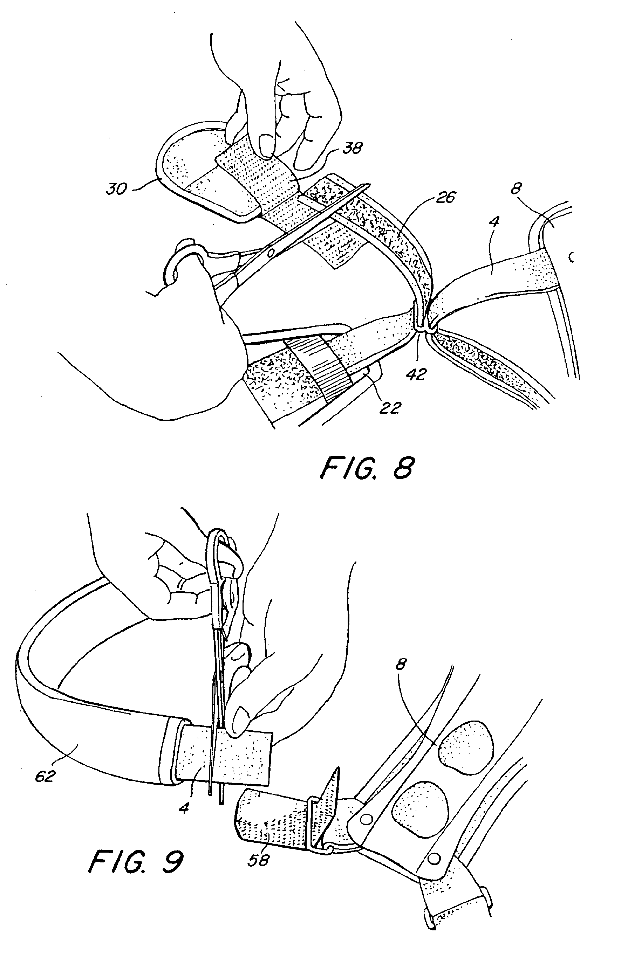

As can be appreciated, the length of the respective strap members 4 and 6 can be appropriately adjusted by an orthotist, as seen in Figure 9, by cutting the ends of the straps which have an exterior loop fabric texture and then capturing the cut end within the respective upper adjustment straps 58 and 60. Thus, as is shown in Figure 9, the free end of the flexible strap member 4 is cut to size for a particular patient and then secured in a sandwich like manner by the hook material provided on and between the upper adjustment straps 58.

As can be seen in Figure 8, a similar size adjustment procedure can be performed by an orthotist in cutting, for example, the connecting strap 26 at an end adjacent the fastening pad 30. The abdomen adjustment straps 38 and 40 also have a hook material on their interior surfaces and adhere to the napped fabric on the exterior of the connecting straps 26 and 28, respectively, when secured in a sandwich manner over the ends of the adjustment straps. Thus, the present invention permits two areas of adjustment by cutting or removing unwanted lengths of the respective fastening strap members 4 and 6 and the respective connecting straps 26 and 28.

The spinal frame member 74 has a cross sectional curved neck portion 78 to provide rigidity and strength to the reduced width at the top of the spinal frame member 74 while also curved along a longitudinal axis to accommodate the curve of the spine. A series of holes 82 can be provided for reducing weight. The lower triangular base 76 also has an elongated central slot 80, also to reduce weight. The lower tip of base 76 can be flared outward at the base of the spine. The resilient spinal pad 84 has a napped fabric on one side specifically designed for adhering to a hook material, for example of a Velcro® brand. Swatches of Velcro® hook material 86 can be appropriately adhered along an interior surface of the spinal frame 74 as shown in dotted lines in Figure 2, as an illustration. The other side of the resilient spinal pad 84 can have a pocked or apertured textured surface configuration to assist in aerating the length of the resilient spinal pad 84 that will bear against a user. The spinal pad 84 is removable from the hook swatches 86 and washable for hygienic reasons.

After an orthotist has appropriately sized the length of the pair of flexible strap members 4 and 6 and likewise the length of the connecting straps 26 and 28 as shown in Figures 8 and 9, the spinal orthosis 2 can then be easily donned by a user with limited mobility. The upper major loops formed by the flexible strap members 4 and 6 will be encompassed with the shoulder tube pads 62 and 64. The user can place their arms through these major loops so that the shoulder tube pads 62 and 64 are appropriately placed across the user's shoulders, as shown for example, in Figure 6. The user can then close the respective abdomen support members 22 and 24 so that the loop and hook material can adhere the abdomen support members 22 and 24 together while the straps 4 and 6 are still in a relatively loose condition.

As shown in Figure 7, the user can either grasp the fastening pads 30 and 32 and tighten the spinal orthosis against the spine as shown, for example, in Figure 5, or can insert her/his fingers into the respective pockets 34 and 36 on the respective fastening pads 30 and 32 and by extending her/his arms, tighten the brace 2 to the spine of the user while pulling the flexible strap members 4 and 6. By pressing the hook surface of the fastening pads 30 and 32 against the nap material of the abdomen support member or the exterior nap surface of a corresponding fastening pad, the tightened position is then retained for wearing purposes.

In summary, the spinal orthosis can position a posterior spinal member 8 so that its length extends across the thoracic to the adjacent coccyx of the user's spine. The arrangement of the fastening straps 4 and 6 provide not only an adjustable set of shoulder straps that can be easily donned by a user, but further contribute to the easy fastening of the flexible abdominal support members 24 and 22 together wherein extensions of the fastening pads 30 and 32 can removably secure the brace in position on the user's spine while specifically tightening the shoulder straps to draw the user's shoulders back against the posterior spinal member 8.

## Claims

1. A spinal orthosis that is configured to be adjustably mounted to enable a user to apply extension and compression forces when tightening the spinal orthosis onto the user while being easily released to permit freedom of movement by the user, comprising:
a posterior spinal member (8) configured to support a user's spine; and
a pair of elongated flexible strap members (4, 6), each fastened at one end of the respective strap member (4, 6) on an upper portion of the posterior spinal member (8) and each fastened at the other end of the respective strap member (4, 6) on a lower portion of the posterior spinal member (8), the respective strap members (4, 6) extending on opposite sides of the posterior spinal member (8) and having a sufficient length to provide shoulder straps for the user;
the spinal orthosis being **characterized by**:
a pair of attachment members (14, 16, 18, 20, 42, 44) located on the posterior spinal member (8) configured to enable a sliding movement for each flexible strap member (4, 6) through a corresponding attachment member (14, 16, 18, 20, 42, 44) to tighten the posterior spinal member (8) about the user's spine, one of each attachment member (14, 16, 18, 20, 42, 44) is located along a respective opposite side of the posterior spinal member (8) for enabling a respective flexible strap member (4, 6) to form an upper shoulder strap loop adjacent the upper portion of the posterior spinal member (8) and for providing a smaller loop than the shoulder strap loop adjacent the lower portion of the posterior spinal member (8); and
a pair of flexible abdomen support members (22, 24), each having a connecting strap (26, 28) attached to an abdomen support member (22, 24) at one end of the connecting strap (26, 28) and slidably connected to a respective smaller loop of the flexible strap member(4, 6), wherein the flexible abdomen support members (22, 24) are configured to adhere together by the user to initially secure the spinal orthosis on the user and the other end of each of the connecting straps (26, 28) is configured to be pulled by the user to tighten the shoulder strap loop and the smaller loop, by applying extension and compression forces on the user, and to be fastened, relative to the spinal orthosis, to complete securement of the spinal orthosis on the user.

2. The spinal orthosis of claim 1, wherein the flexible abdomen support members (22, 24) adhere together when overlapped.

3. The spinal orthosis of claim 2, wherein the flexible abdomen support members (22, 24) have one of a nap material and a hook material on a surface for adhering together.

4. The spinal orthosis of claim 1, wherein each connecting strap (26, 28) has a fastening pad (30, 32) at one end and an abdomen support member (22, 24) at the other end.

5. The spinal orthosis of claim 4, wherein the fastening pad (30, 32) secures to a surface of one of the flexible abdomen support members (22, 24).

6. The spinal orthosis of claim 5, wherein the flexible abdomen support member (22, 24) has an outer surface with a complementary surface (70, 72) for securement to a fastening pad (30, 32).

7. The spinal orthosis of claim 4, wherein the fastening pad (30, 32) includes a pocket (34, 36) to permit a user's finger and/or fingers to be captured for tightening the connecting strap (26, 28).

8. The spinal orthosis of claim 7, wherein the fastening pad (30, 32) includes a pair of flexible straps (38, 40) that removably adhere to one end of the connecting strap (26, 28).

9. The spinal orthosis of claim 1, wherein a loop fastener (42, 44) connects the connecting strap (26, 28) for sliding movement on the smaller loop of the flexible strap member (4, 6).

10. The spinal orthosis of claim 1, wherein the posterior spinal member (8) is a curved flat metal plate having a triangular base (76) and a narrower curved upper portion (78).

11. The spinal orthosis of claim 10, wherein the posterior spinal member (8) includes strips of hook material (86) and a resilient pad (84) is removably connected to the hook material (86).

12. The spinal orthosis of claim 11, wherein one surface of the resilient pad (84) has a nap material and the opposite surface has an apertured texture to permit air flow.

13. The spinal orthosis of claim 1, wherein the pair of flexible strap members (4, 6) are attached to respectively cantilevered loops (54, 56) at the top of the posterior spinal member (8) and the flexible strap members (4, 6) have a removable strap (58, 60) that secures to both sides of each end of the strap members (4, 6) to enable adjustment and fit of a length of the strap members (4, 6) to the size of the user with the removable strap (58, 60) captured in the cantilevered loop (54, 56).

## Patentansprüche

1. Eine Wirbelsäulen-Orthese, die konfiguriert ist, um einstellbar angebracht zu sein, um es einem Benutzer zu ermöglichen, beim Festziehen der Wirbelsäulen-Orthese Streck-und Presskräfte auf den Benutzer aufzubringen, wobei sie einfach gelöst werden kann, um eine Bewegungsfreiheit des Benutzers zuzulassen, aufweisend:
ein Posteriores-Wirbelsäulen-Element (8), welches konfiguriert ist, um eine Wirbelsäule des Benutzers zu stützen, und
ein Paar von lang gestreckten, flexiblen Riemenelementen (4, 6), wobei jedes an einem Ende des jeweiligen Riemenelements (4, 6) an einem oberen Abschnitt des Posterioren-Wirbelsäulen-Elements (8) befestigt ist und jedes am anderen Ende des jeweiligen Riemenelements (4, 6) an einem unteren Abschnitt des Posterioren-Wirbelsäulen-Elements (8) befestigt ist, wobei sich die jeweiligen Riemenelemente (4, 6) an entgegengesetzten Seiten des Posterioren-Wirbelsäulen-Elements (8) erstrecken und eine ausreichende Länge haben, um Schulterriemen für den Benutzer bereitzustellen, wobei die Wirbelsäulen-Orthese **gekennzeichnet ist durch**:
ein Paar von Halterungselementen (14, 16, 18, 20, 42, 44), welche am Posterioren-Wirbelsäulen-Element (8) angeordnet sind, und welche konfiguriert sind, um eine Verschiebebewegung für jedes flexible Riemenelement (4, 6) **durch** ein korrespondierendes Halterungselement (14, 16, 18, 20, 42, 44) zu ermöglichen, um das Posteriore-Wirbelsäulen-Element (8) gegen die Wirbelsäule des Benutzers festzuziehen, wobei jeweils eines von jedem Halterungselement (14, 16, 18, 20, 42, 44) entlang einer jeweiligen entgegengesetzten Seite des Posterioren-Wirbelsäulen-Elements (8) angeordnet ist, um es einem jeweiligen flexiblen Riemenelement (4, 6) zu ermöglichen, eine obere Schulterriemenschlaufe auszubilden, welche zu dem oberen Abschnitt des Posterioren-Wirbelsäulen-Elements (8) benachbart ist, und um eine kleinere Schlaufe als die Schulterriemenschlaufe benachbart zu dem unteren Abschnitt des Posterioren-Wirbelsäulen-Elements (8) bereitzustellen, und
ein Paar von flexiblen Unterleib-Abstütz-Elementen (22, 24), wobei jedes einen Verbindungsriemen (26, 28) hat, welcher an einem Unterleib-Abstütz-Element (22, 24) an einem Ende des Verbindungsriemens (26, 28) angebracht ist und verschiebbar mit einer jeweiligen kleineren Schlaufe des flexiblen Riemenelements (4, 6) verbunden ist, wobei die flexiblen Unterleib-Abstütz-Elemente (22, 24) konfiguriert sind, um mittels des Benutzers aneinander angehaftet zu werden, um die Wirbelsäulen-Orthese am Benutzer anfänglich zu fixieren, wobei das andere Ende eines jeden der Verbindungsriemen (26, 28) konfiguriert ist, um vom Benutzer gezogen zu werden, um die Schulterriemenschlaufe und die kleinere Schlaufe festzuziehen, bei Aufbringen von Streck- und Presskräften auf den Benutzer, und um bezüglich der Wirbelsäulen-Orthese befestigt zu werden, um das Fixieren der Wirbelsäulen-Orthese am Benutzer zu vollenden.

2. Die Wirbelsäulen-Orthese gemäß Anspruch 1, wobei die flexiblen Unterleib-Abstütz-Elemente (22, 24) aneinander anhaften, wenn sie überlappend sind.

3. Die Wirbelsäulen-Orthese gemäß Anspruch 2, wobei die flexiblen Unterleib-Abstütz-Elemente (22, 24) jeweils eines von einem Härchenmaterial und einem Hakenmaterial auf einer Fläche zum aneinander Anhaften haben.

4. Die Wirbelsäulen-Orthese gemäß Anspruch 1, wobei jeder Verbindungsriemen (26, 28) eine Befestigungsunterlage (30, 32) an einem Ende und ein Unterleib-Abstütz-Element (22, 24) am anderen Ende hat.

5. Die Wirbelsäulen-Orthese gemäß Anspruch 4, wobei die Befestigungsunterlage (30, 32) an einer Fläche von einem der flexiblen Unterleib-Abstütz-Elemente (22, 24) fixierbar ist.

6. Die Wirbelsäulen-Orthese gemäß Anspruch 5, wobei das flexible Unterleib-Abstütz-Element (22, 24) eine äußere Fläche mit einer komplementären Fläche (70, 72) hat zum Fixieren an einer Befestigungsunterlage (30, 32).

7. Die Wirbelsäulen-Orthese gemäß Anspruch 4, wobei die Befestigungsunterlage (30, 32) eine Tasche (34, 36) aufweist, um es einem Finger und/oder Fingern des Benutzers zu erlauben, zum Festziehen des Verbindungsriemens (26, 28) darin Halt zu finden.

8. Die Wirbelsäulen-Orthese gemäß Anspruch 7, wobei die Befestigungsunterlage (30, 32) ein Paar von flexiblen Riemen (38, 40) aufweist, die lösbar an einem Ende des Verbindungsriemens (26, 28) anhaften.

9. Die Wirbelsäulen-Orthese gemäß Anspruch 1, wobei ein Schlaufen-Befestigungsmittel (42, 44) den Verbindungsriemen (26, 28) zum Verschiebe-Bewegen mit der kleineren Schlaufe des flexiblen Riemenelements (4, 6) verbindet.

10. Die Wirbelsäulen-Orthese gemäß Anspruch 1, wobei das Posteriore-Wirbelsäulen-Element (8) eine gekrümmte, flache Metallplatte ist, welche eine dreieckige Basis (76) und einen schmaleren, gekrümmten oberen Abschnitt (78) hat.

11. Die Wirbelsäulen-Orthese gemäß Anspruch 10, wobei das Posteriore-Wirbelsäulen-Element (8) Streifen von Hakenmaterial (86) und eine elastische Unterlage (84) aufweist, welche lösbar mit dem Hakenmaterial (86) verbunden ist.

12. Die Wirbelsäulen-Orthese gemäß Anspruch 11, wobei eine Fläche der elastischen Unterlage (84) ein Härchenmaterial hat und die entgegengesetzte Fläche eine gelochte Textur hat, um einen Luftfluss zuzulassen.

13. Die Wirbelsäulen-Orthese gemäß Anspruch 1, wobei das Paar von flexiblen Riemenelementen (4, 6) an jeweiligen frei auskragenden Schlaufen (54, 56) oben am Posterioren-Wirbelsäulen-Element (8) angebracht ist und die flexiblen Riemenelemente (4, 6) einen lösbaren Riemen (58, 60) haben, der an beiden Seiten des jeweiligen Endes der Riemenelemente (4, 6) fixiert ist, um die Einstellbarkeit und die Anpassung einer Länge der Riemenelemente (4, 6) an die Größe des Benutzers zu ermöglichen, wobei der lösbare Riemen (58, 60) in den jeweiligen frei auskragenden Schlaufen (54, 56) erfasst ist.

## Revendications

1. Orthèse vertébrale configurée pour être montée de manière réglable pour permettre à un utilisateur d'appliquer des forces d'extension et de compression lors du serrage de l'orthèse vertébrale sur l'utilisateur tout en étant facilement enlevée pour permettre la liberté de mouvement, par l'utilisateur, comprenant :
un élément vertébral postérieur (8) configuré pour supporter la colonne vertébrale d'un utilisateur ; et
une paire d'éléments de sangle flexibles allongés (4, 6), chacun fixé à une extrémité de l'élément de sangle (4, 6) respectif sur une partie supérieure de l'élément vertébral postérieur (8) et chacun fixé à l'autre extrémité de l'élément de sangle (4, 6) respectif sur une partie inférieure de l'élément vertébral postérieur (8), les éléments de sangle (4, 6) respectifs s'étendant sur des côtés opposés de l'élément vertébral postérieur (8) et ayant une longueur suffisante pour fournir des bretelles pour l'utilisateur ;
l'orthèse vertébrale étant **caractérisée par** :
une paire d'éléments de fixation (14, 16, 18, 20, 42, 44) positionnés sur l'élément vertébral postérieur (8) configuré pour permettre un mouvement coulissant pour chaque élément de sangle flexible (4, 6) par le biais d'un élément de fixation (14, 16, 18, 20, 42, 44) correspondant afin de serrer l'élément vertébral postérieur (8) autour de la colonne vertébrale de l'utilisateur, chacun des éléments de fixation (14, 16, 18, 20, 42, 44) est positionné le long d'un côté opposé respectif de l'élément vertébral postérieur (8) pour permettre à un élément de sangle flexible (4, 6) respectif de former une boucle de bretelle supérieure adjacente à la partie supérieure de l'élément vertébral postérieur (8) et pour fournir une plus petite boucle que la boucle de bretelle adjacente à la partie inférieure de l'élément vertébral postérieur (8) ; et
une paire d'éléments de maintien abdominal flexibles (22, 24), ayant chacun une sangle de raccordement (26, 28) fixée sur un élément de maintien abdominal (22, 24) à une extrémité de la sangle de raccordement (26, 28) et raccordée de manière coulissante à une plus petite boucle respective de l'élément de sangle flexible (4, 6), dans laquelle les éléments de maintien abdominal flexibles (22, 24) sont configurés pour être fixés ensemble par l'utilisateur afin de fixer initialement l'orthèse vertébrale sur l'utilisateur et l'autre extrémité de chacune des sangles de raccordement (26, 28) est configurée pour être tirée par l'utilisateur afin de serrer la boucle de bretelle et la plus petite boucle, en appliquant des forces d'extension et de compression sur l'utilisateur, et être fixée, par rapport à l'orthèse vertébrale, pour achever la fixation de l'orthèse vertébrale sur l'utilisateur.

2. Orthèse vertébrale selon la revendication 1, dans laquelle les éléments de maintien abdominal flexibles (22, 24) se fixent ensemble lorsqu'ils se chevauchent.

3. Orthèse vertébrale selon la revendication 2, dans laquelle les éléments de maintien abdominal flexibles (22, 24) ont l'un parmi un matériau à poils et un matériau à crochets sur une surface pour adhérer l'un par rapport à l'autre.

4. Orthèse vertébrale selon la revendication 1, dans laquelle chaque sangle de raccordement (26, 28) a un coussinet de fixation (30, 32) à une extrémité et un élément de maintien abdominal (22, 24) à l'autre extrémité.

5. Orthèse vertébrale selon la revendication 4, dans laquelle le coussinet de fixation (30, 32) se fixe sur une surface de l'un des éléments de maintien abdominal flexibles (22, 24).

6. Orthèse vertébrale selon la revendication 5, dans laquelle l'élément de maintien abdominal flexible (22, 24) a une surface externe avec une surface complémentaire (70, 72) pour la fixation sur un coussinet de fixation (30, 32).

7. Orthèse vertébrale selon la revendication 4, dans laquelle le coussinet de fixation (30, 32) comprend une poche (34, 36) pour permettre au doigt et/ou aux doigts d'un utilisateur d'être pris pour serrer la sangle de raccordement (26, 28).

8. Orthèse vertébrale selon la revendication 7, dans laquelle le coussinet de fixation (30, 32) comprend une paire de sangles flexibles (38, 40) qui adhèrent de manière détachable à une extrémité de la sangle de raccordement (26, 28).

9. Orthèse vertébrale selon la revendication 1, dans laquelle une fixation à boucle (42, 44) raccorde la sangle de raccordement (26, 28) pour le mouvement coulissant sur la plus petite boucle de l'élément de sangle flexible (4, 6).

10. Orthèse vertébrale selon la revendication 1, dans laquelle l'élément vertébral postérieur (8) est une plaque métallique plate incurvée ayant une base triangulaire (76) et une partie supérieure (78) incurvée plus étroite.

11. Orthèse vertébrale selon la revendication 10, dans laquelle l'élément vertébral postérieur (8) comprend des bandes de matériau à crochets (86) et un coussinet élastique (84) est raccordé de manière détachable au matériau à crochets (86).

12. Orthèse vertébrale selon la revendication 11, dans laquelle une surface du coussinet élastique (84) a un matériau à poils et la surface opposée a une texture ouverte pour permettre l'écoulement de l'air.

13. Orthèse vertébrale selon la revendication 1, dans laquelle la paire d'éléments de sangle flexibles (4, 6) sont fixés aux boucles (54, 56) respectivement en porte-à-faux au sommet de l'élément vertébral postérieur (8) et les éléments de sangle flexibles (4, 6) ont une sangle amovible (58, 60) qui se fixe sur les deux côtés de chaque extrémité des éléments de sangle (4, 6) pour permettre le réglage ou l'ajustement d'une longueur des éléments de sangle (4, 6) à la taille de l'utilisateur avec la sangle (58, 60) amovible prise dans la boucle en porte-à-faux (54, 56).
